Europäisches Patentamt

(19) European Patent Office

Office européen des brevets

(11) Numéro de publication : **0 346 184 B1**

(12) **FASCICULE DE BREVET EUROPEEN**

(45) Date de publication du fascicule du brevet :
04.12.91 Bulletin 91/49

(51) Int. Cl.⁵ : **B01J 23/94,** B01J 37/24, C07C 2/24

(21) Numéro de dépôt : 89401471.1

(22) Date de dépôt : 30.05.89

(54) **Procédé de régénération de catalyseurs de dimérisation d'oléfines renfermant du chlore et du nickel déposés sur alumine.**

(30) Priorité : 06.06.88 FR 8807599

(43) Date de publication de la demande :
13.12.89 Bulletin 89/50

(45) Mention de la délivrance du brevet :
04.12.91 Bulletin 91/49

(84) Etats contractants désignés :
BE DE GB IT NL

(56) Documents cités :
FR-A- 2 329 347
US-A- 2 968 631
US-A- 3 047 491

(73) Titulaire : **INSTITUT FRANCAIS DU PETROLE**
**4, Avenue de Bois-Préau**
**F-92502 Rueil-Malmaison (FR)**

(72) Inventeur : **Hugues, François**
**2, Av. Félix Faure**
**F-92000 Nanterre (FR)**
Inventeur : **Commereuc, Dominique**
**32, rue Abel Vacher**
**F-92190 Meudon (FR)**
Inventeur : **Chauvin, Yves**
**64, avenue du Général Leclerc**
**F-78230 Le Pecq (FR)**
Inventeur : **Saussine, Lucien**
**2, place des Frères Tissandier**
**F-78290 Croissy sur Seine (FR)**
Inventeur : **Bournonville, Jean-Paul**
**43, rue des Groues Vauréal**
**F-95000 Cergy Pontoise (FR)**

EP 0 346 184 B1

Jouve, 18, rue Saint-Denis, 75001 PARIS

## Description

La présente invention concerne un procédé de régénération de catalyseurs usagés de dimérisation d'oléfines renfermant du chlore et du nickel, dont les proportions sont de préférence non stoechiométriques, déposés sur alumine.

La dimérisation et l'oligomérisation des oléfines légères à l'aide de catalyseurs consistant en divers composés du nickel déposés sur des supports minéraux sont bien connues.

Le brevet US-A 2794842 décrit la polymérisation de l'éthylène en présence de sulfate de nickel déposé sur silice-alumine.

FEL'DBLYUM et al. décrivent, dans le "Zhurnal Organicheskoi Khimii", volume 8, numéro 3, pages 650 et 651, mars 1972, la dimérisation de l'éthylène et du propylène sur un catalyseur composé de chlorure de nickel déposé sur silice-alumine ; les auteurs y indiquent que l'activité élevée du catalyseur n'est maintenue que pendant 5,5 heures de fonctionnement en continu.

Dans le brevet US-A 3959400, de l'alumine gamma imprégnée avec du chlorure de nickel conduit à un catalyseur de dimérisation du propylène réellement actif et sélectif pendant une durée de 8,5 jours seulement ; l'activité de ce catalyseur après régénération par chauffage sous atmosphère d'azote à 510°C baisse fortement : ainsi, contrairement aux catalyseurs renfermant du sulfate de nickel déposé sur alumine dont l'activité est au moins partiellement restaurée après régénération par simple calcination, les catalyseurs renfermant du chlorure de nickel déposé sur alumine perdent une partie de leur activité lors de leur régénération par chauffage.

La présente invention remédie en particulier à cet inconvénient majeur que constitue la diminution d'activité d'un catalyseur renfermant du chlore et du nickel déposés sur alumine lors de sa régénération.

Son objet est un procédé de régénération d'un catalyseur usagé de dimérisation d'oléfines renfermant du chlore et du nickel déposés sur alumine, cette régénération s'accompagnant d'une augmentation de l'activité dudit catalyseur, catalyseur qui peut retrouver après sa régénération selon l'invention sensiblement son activité initiale (c'est-à-dire son activité avant son utilisation dans la réaction de dimérisation des oléfines).

Selon l'invention, un catalyseur renfermant du chlore et du nickel déposés sur alumine est, après avoir été utilisé dans une réaction de dimérisation d'oléfines, régénéré par chauffage sous atmosphère chlorante et oxydante (régénération oxychlorante).

L'oléfine employée dans la réaction de dimérisation dans laquelle intervient le catalyseur renfermant du chlore et du nickel déposés sur alumine peut notamment être l'éthylène, le propylène ou l'un des isomères des butènes. Cette oléfine peut être mise en oeuvre à l'état pur ou en mélange avec un ou plusieurs composés ne réagissant pas sur le catalyseur dans les conditions utilisées, tels que les hydrocarbures saturés cycliques ou acycliques, en particulier ceux ayant de 2 à 10 atomes de carbone. La concentration de l'oléfine dans le mélange peut être de 5 à 100%, de préférence de 10 à 50% en poids. On préfère utiliser une charge sensiblement exempte d'eau, ce qui peut être réalisé par exemple en séchant la charge au préalable sur tamis moléculaire 3 A : une teneur pondérale en eau inférieure à 100 ppm et plus particulièrement inférieure à 10 ppm est préférée.

Le catalyseur renfermant du chlore et du nickel peut être préparé selon toute technique connue, en particulier par imprégnation : le mode opératoire préféré consiste en l'imprégnation de l'alumine, préalablement séchée, avec une solution contenant du chlorure de nickel ; cette imprégnation peut éventuellement être effectuée en utilisant un volume de liquide au maximum égal au volume poreux du support (c'est-à-dire de l'alumine) ou en utilisant un excès de solution. Après la phase d'imprégnation, le catalyseur est séché et calciné. Le séchage peut être effectué par exemple par passage d'un courant d'air sec, dont la teneur pondérale en eau est de préférence inférieure à 100 ppm, à travers le solide, à 100-150°C pendant 0,5 à 6 heures. La calcination peut être réalisée par chauffage en atmosphère non réductrice, à des températures comprises entre 300 et 800°C, de préférence entre 400 et 600°C, pour une durée comprise par exemple entre 0,5 et 12 heures. Après calcination, le catalyseur est utilisable pour effectuer la réaction de dimérisation.

Le solvant utilisé dans la préparation du catalyseur peut être par exemple l'eau.

L'alumine est habituellement une alumine gamma ou êta. L'alumine présente avantageusement une surface spécifique d'au moins 20 $m^2 \cdot g^{-1}$ et un volume poreux d'au moins 0,1 $cm^3 \cdot g^{-1}$. On peut par exemple l'utiliser sous forme de billes ou d'extrudés.

Le catalyseur possède initialement, c'est-à-dire avant d'être utilisé dans la réaction de dimérisation (donc avant d'être au moins partiellement désactivé), une teneur en nickel comprise en général entre 0,5 et 15% en poids, et de préférence entre 1,5 et 6%, et une teneur en chlore comprise par exemple entre 0,4 et 15% en poids, et de préférence entre 1,3 et 7%.

La réaction de dimérisation est de préférence effectuée en continu, le catalyseur étant disposé en lit fixe dans un tube et étant traversé par le mélange réactionnel. La pression est maintenue de telle façon que le mélange réactionnel soit à l'état liquide ; cette pression dépend donc de la température de réaction, de l'oléfine employée et de sa concentration dans le mélange. La vitesse volumique horaire est habituellement choisie entre 0,1 et 10 $h^{-1}$, selon les conversions désirées, pour un volume de catalyseur égal à l'unité. La température est utilement choisie entre 10 et 150°C ; il est préférable de la maintenir entre 30 et 60°C.

Pendant la réaction de dimérisation on constate que l'activité du catalyseur diminue. Le procédé, selon l'invention, permet de régénérer ce catalyseur, au moins partiellement désactivé, renfermant initialement du chlore et du nickel déposés sur alumine, et de lui restituer sensiblement son activité initiale. Le procédé de régénération du catalyseur selon l'invention comporte les étapes successives suivantes :

a) on sèche le catalyseur sous courant d'au moins un gaz choisi dans le groupe constitué par un gaz contenant de l'oxygène moléculaire (l'air par exemple) et un gaz inerte, à une température comprise généralement entre environ 5 et 70°C, de préférence entre environ 15 et 70°C, de façon à éliminer, de préférence, au moins la majorité (au moins 50%) des hydrocarbures encore présents sur ledit catalyseur et, de manière encore plus préférée, au moins la majeure partie (environ 80% au moins) de ces hydrocarbures ;

b) on calcine le catalyseur issu de l'étape a) par chauffage en atmosphère non réductrice, à une température généralement comprise entre environ 300 et 800°C, de préférence entre 400 et 600°C ;

c) on chauffe le catalyseur issu de l'étape b) sous atmosphère oxydante et chlorante, à une température habituellement comprise entre environ 25 et 750°C, de préférence entre 300 et 750°C ;

d) on calcine le catalyseur issu de l'étape c) par chauffage en atmosphère non réductrice, à une température généralement comprise entre environ 300 et 800°C, de préférence entre 400 et 600°C, la température de l'étape d) étant de préférence supérieure d'au moins 50°C à la température de l'étape c) ;

e) éventuellement, on refroidit le catalyseur issu de l'étape d) jusqu'à la température adéquate, par exemple jusqu'à la température ambiante (généralement comprise entre 10 et 30°C).

Le procédé de régénération comprenant les étapes a) à d), plus éventuellement l'étape e), peut être répété plusieurs fois.

La durée de séchage, c'est-à-dire la durée de l'étape a), est habituellement comprise entre environ 0,5 et 7 heures. Le terme gaz inerte désigne dans la présente invention tout gaz qui ne réagit pas avec le catalyseur, tel que par exemple l'azote, l'hélium, l'argon ou des mélanges de ces gaz. La teneur pondérale en eau du courant de gaz (dans l'étape a)) est, de préférence, inférieure à 100 ppm.

La durée de l'étape b) et la durée de l'étape d) sont utilement comprises entre environ 0,5 et 12 heures.

L'atmosphère non réductrice dans l'étape b) et l'étape d) est généralement à base d'un courant d'un gaz contenant de l'oxygène moléculaire (par exemple de l'air sec), dont la teneur en eau est avantageusement inférieure à 100 ppm en poids.

Le catalyseur issu de l'étape b) est chauffé sous atmosphère oxydante et chlorante pendant, de préférence, environ 0,5 à 6 heures (étape c)).

Dans cette étape c), l'atmosphère oxydante et chlorante est généralement un courant gazeux contenant un mélange d'au moins un gaz renfermant de l'oxygène moléculaire (par exemple de l'air) et d'au moins un composé chloré (c'est-à-dire un composé minéral ou organique susceptible de libérer du chlore). Le composé chloré (ou agent chlorant) utilisé dans l'étape c) peut être choisi notamment dans le groupe formé par le chlore, le chlorure d'hydrogène, le tétrachlorure de carbone, le chlorure de méthyle, le dichloroéthane, le dichloropropane -1,2, le chlorure de méthylène et le chloroforme et, éventuellement, les mélanges d'au moins deux quelconques de ces constituants.

La teneur pondérale en eau du gaz contenant de l'oxygène moléculaire dans l'étape c) dépend du composé chloré utilisé ; elle est en général avantageusement comprise entre 0,01 et 0,5%.

Le composé chloré est employé en une quantité représentant, habituellement, au total de 1 à 10% et de préférence de 1,5 à 5% en poids calculé en poids de chlore par rapport au poids de catalyseur (issu de l'étape b)).

Le débit horaire du gaz contenant de l'oxygène moléculaire dans l'étape c) est généralement de l'ordre de 0,1 à 10 litres et de préférence de 0,5 à 2,5 litres par gramme de catalyseur.

Dans l'étape c), on peut préférentiellement placer en amont du catalyseur issu de l'étape b), par rapport au courant gazeux, une charge de catalyseur frais (c'est-à-dire non désactivé) de même nature (renfermant du chlore et du nickel déposés sur alumine) qui servira de catalyseur "tampon" et permettra ainsi d'obtenir une répartition encore plus homogène du chlore sur le catalyseur issu de l'étape b). Le rapport pondéral entre la quantité de catalyseur "tampon" et la quantité de catalyseur à traiter est en général choisi entre 0,01 et 1 et de préférence entre 0,1 et 1.

La mesure du taux de chlore sur le catalyseur, qui renseigne sur l'activité de celui-ci bien que la relation entre activité et taux de chlore présent ne soit pas clairement établie, peut être effectuée notamment par fluorescence X sur le solide brut (ou catalyseur brut) ou par dosage potentiométrique après extraction du chlore déposé sur ledit solide.

Le catalyseur régénéré selon le procédé de la présente invention retrouve sensiblement son activité initiale.

La présente invention a également pour objet un procédé de dimérisation d'au moins un hydrocarbure mono-oléfinique comprenant :

1) une première étape, en phase liquide, dans laquelle au moins un catalyseur de dimérisation d'oléfines renfermant du chlore et du nickel (dont les proportions sont de préférence non-stoechiométriques) déposés sur alumine, la teneur pondérale en nickel dudit catalyseur étant en général comprise entre 0,5 et 15% et la teneur pondérale en chlore dudit catalyseur étant habituellement comprise entre 0,4 et 15%, est mis en contact avec au moins un hydrocarbure mono-oléfinique, choisi par exemple dans le groupe formé par l'éthylène, le propylène, les isomères des butènes et les mélanges d'au moins deux de ces constituants, dans des conditions de dimérisation, ladite étape étant poursuivie jusqu'à ce que le catalyseur ait perdu au moins en partie son activité initiale, c'est-à-dire, en général, jusqu'à ce qu'il ait perdu 10 à 60%, de préférence 20 à 50%, de son activité initiale ;

2) une deuxième étape dans laquelle le catalyseur issu de l'étape 1) est soumis à une régénération, ladite régénération étant effectuée selon le procédé de l'invention décrit ci-avant (cette régénération ayant en général lieu au moins en partie dans au moins une enceinte différente de celle de la réaction de dimérisation) ;

3) une troisième étape dans laquelle le catalyseur issu de l'étape 2) est mis en contact avec au moins un hydrocarbure mono-oléfinique dans des conditions de dimérisation, c'est-à-dire est renvoyé à l'étape 1) (par exemple le catalyseur peut être utilisé pour dimériser une charge différente de celle utilisée à l'étape 1)).

Le cycle ainsi décrit comprenant une période de dimérisation, une période de régénération, puis une nouvelle période de dimérisation, peut être répétée plusieurs fois.

Les exemples suivants illustrent l'invention (sans en limiter la portée).

## EXEMPLE 1

325 grammes d'alumine gamma (de surface spécifique 238 $m^2 \cdot g^{-1}$ et de volume poreux 0,52 $cm^3 \cdot g^{-1}$), sous forme de billes de diamètre 0,2-0,4 mm, préalablement calcinée sous air sec à 500°C pendant 3 heures, sont imprégnés à sec, à 25°C, avec une solution contenant 55 grammes de $NiCl_2$, $6H_2O$. Après séchage sous air à 150°C pendant 2 heures, le solide est calciné sous air sec à 500°C pendant 3 heures. L'analyse du solide par fluorescence X à ce stade indique qu'il contient 4,0% en poids de nickel et 2,27% en poids de chlore.

## EXEMPLE 2

49 grammes du catalyseur préparé dans l'exemple 1 sont placés dans un tube en acier inoxydable servant de réacteur. Une solution, maintenue sous une pression de 4 bars et contenant 10% en poids de propylène dans le n-heptane, est envoyée à travers le lit catalytique, maintenu à 40°C, avec une vitesse volumique horaire de 0,81 $h^{-1}$. A la sortie du réacteur, l'effluent est analysé par chromatographie en phase vapeur ; l'analyse indique que la conversion du propylène est de 97,5% et la sélectivité en hexènes est de 80%.

## EXEMPLE 3

Le catalyseur de l'exemple 2 est séché sous courant d'air sec à 20°C (étape a) selon l'invention), puis calciné sous air sec à 500°C pendant 3 heures (étape b) selon l'invention). L'analyse du solide par fluorescence X à ce stade indique qu'il ne contient plus que 1,94% en poids de chlore. Ce catalyseur solide est alors soumis à un test de dimérisation du propylène dans les mêmes conditions que celles de l'exemple 2. La conversion du propylène est de 87%. Les deux étapes a) et b) du procédé selon l'invention ne suffisent donc pas à régénérer correctement le catalyseur.

## EXEMPLE 4

Le catalyseur désactivé de l'exemple 3 est soumis à une opération de séchage sous courant d'air sec à 20°C (étape a) selon l'invention), puis calciné sous air sec à 500°C pendant 3 heures (étape b) selon l'invention). L'analyse du solide indique que le taux de chlore est de 1,65% en poids. Le catalyseur est alors chauffé à 400°C et traversé par un courant d'air, dont le débit horaire est d'environ 50 litres (c'est-à-dire environ 1 litre par gramme de catalyseur), dans lequel on a injecté en continu 1,1 gramme de tétrachlorure de carbone représentant au total 2,1% en poids de chlore par rapport au poids de catalyseur. La durée du traitement est de 100 minutes environ (étape c) du procédé de régénération). Lorsque l'injection de tétrachlorure de carbone est terminée, la température est montée jusqu'à 500°C sous courant d'air sec et maintenue ainsi pendant 3 heures (étape d) du procédé). Après refroidissement (étape e) du procédé) jusqu'à température ambiante (environ 20°C), le catalyseur solide est analysé par fluorescence X ; on constate qu'il contient 3,51% en poids de chlore. Le catalyseur est alors soumis à un test de dimérisation du propylène dans les mêmes conditions que celles de l'exemple 2 : la conversion du propylène est de 96,5% et la sélectivité en hexènes de 76%.

## EXEMPLE 5

49 grammes du catalyseur préparé dans l'exemple 1 sont traités et testés comme dans l'exemple 2 puis dans l'exemple 3.

Ce catalyseur désactivé (identique à celui de l'exemple 3) est alors soumis à une opération de séchage sous courant d'air sec à 20°C (étape **a)** selon l'invention), puis calciné sous air sec à 500°C pendant 3 heures (étape **b)** selon l'invention). Il est ensuite transféré dans un tube en verre pyrex dans lequel est placé, en amont du lit catalytique (c'est-à-dire du catalyseur identique à celui de l'exemple 3), 45 grammes de catalyseur neuf préparé comme dans l'exemple 1 et qui joue le rôle de catalyseur tampon. Puis, l'ensemble des deux masses catalytiques est chauffé à 400°C et traversé par un courant d'air, dont le débit horaire est de l'ordre de 100 litres (soit environ 1 litre par gramme de catalyseur), dans lequel on a injecté en continu 2,1 grammes de tétrachlorure de carbone représentant au total 2,1% en poids de chlore par rapport au poids total de catalyseur. La durée du traitement est d'environ 100 minutes (étape **c)** selon l'invention). Lorsque l'injection de tétrachlorure de carbone est terminée, la température est montée jusqu'à 500°C sous courant d'air sec et maintenue ainsi pendant 3 heures (étape **d)** du procédé de régénération). Après refroidissement jusqu'à 20°C (étape **e)** du procédé), le solide est analysé par fluorescence X ; on constate qu'il contient 2,48% en poids de chlore. Ce catalyseur est alors soumis à un test de dimérisation du propylène dans les mêmes conditions que celles de l'exemple 2 : la conversion du propylène est de 97,4% et la sélectivité en hexènes de 80%.

Le procédé de régénération selon l'invention a ainsi permis de redonner sensiblement au catalyseur renfermant du chlore et du nickel son activité initiale.

**Revendications**

1. Procédé de régénération d'un catalyseur de dimérisation d'oléfines, au moins partiellement désactivé, renfermant du chlore et du nickel déposés sur alumine, caractérisé en ce qu'il comporte les étapes suivantes :

    **a)** on sèche ledit catalyseur sous courant d'au moins un gaz choisi dans le groupe constitué par un gaz contenant de l'oxygène moléculaire et un gaz inerte, à une température comprise entre environ 5 et 70°C ;

    **b)** on calcine le catalyseur issu de l'étape **a)** par chauffage en atmosphère non réductrice, à une température comprise entre environ 300 et 800°C ;

    **c)** on chauffe le catalyseur issu de l'étape **b)** sous atmosphère oxydante et chlorante, à une température comprise entre environ 25 et 750°C ;

    **d)** on calcine le catalyseur issu de l'étape **c)** par chauffage en atmosphère non réductrice, à une température comprise entre environ 300 et 800°C.

2. Procédé selon la revendication 1 caractérisé en ce qu'il comporte en outre après l'étape **d)** une étape dans laquelle le catalyseur issu de ladite étape **d)** est refroidi jusqu'à la température ambiante.

3. Procédé selon l'une des revendications 1 et 2 dans lequel ledit catalyseur possède, avant d'être au moins partiellement désactivé, une teneur pondérale en nickel comprise entre 0,5 et 15% et une teneur pondérale en chlore comprise entre 0,4 et 15%.

4. Procédé selon l'une des revendications 1 et 2 dans lequel ledit catalyseur possède, avant d'être au moins partiellement désactivé, une teneur pondérale en nickel comprise entre 1,5 et 6% et une teneur pondérale en chlore comprise entre 1,3 et 7%.

5. Procédé selon l'une des revendications 1 à 4 dans lequel l'atmosphère non réductrice dans l'étape **b)** et l'étape **d)** est à base d'un courant d'un gaz contenant de l'oxygène moléculaire.

6. Procédé selon l'une des revendications 1 à 5 dans lequel l'atmosphère oxydante et chlorante dans l'étape **c)** est un courant gazeux contenant un mélange d'au moins un gaz renfermant de l'oxygène moléculaire et d'au moins un composé chloré.

7. Procédé selon la revendication 6 dans lequel le composé chloré est choisi dans le groupe formé par le chlore, le chlorure d'hydrogène, le tétrachlorure de carbone, le chlorure de méthyle, le dichloroéthane, le dichloropropane -1,2, le chlorure de méthylène, le chloroforme et les mélanges d'au moins deux de ces constituants.

8. Procédé selon l'une des revendications 6 et 7 caractérisé en ce que, dans l'étape **c)**, on place en amont du catalyseur issu de l'étape **b)**, par rapport au courant gazeux, une charge de catalyseur frais de même nature.

9. Procédé selon la revendication 8 caractérisé en ce que le rapport pondéral entre la quantité dudit catalyseur frais et la quantité du catalyseur à traiter est comprise entre 0,01 et 1.

10. Procédé de dimérisation d'au moins un hydrocarbure mono-oléfinique comprenant :

    **a)** une première période, en phase liquide, dans laquelle au moins un catalyseur de dimérisation renfermant du chlore et du nickel déposés sur alumine est mis en contact avec au moins un hydrocarbure mono-oléfinique dans des conditions de dimérisation, ladite période étant poursuivie jusqu'à ce que le catalyseur ait perdu au moins en partie son activité initiale ;

    **b)** une deuxième période dans laquelle le catalyseur au moins partiellement désactivé issu de la période **a)** est soumis à une régénération, ladite régénération étant effectuée selon le procédé de l'une des revendications 1 à 9 ;

    **c)** une troisième période dans laquelle le catalyseur issu de la période **b)** est renvoyé à la période **a)** de mise en contact avec au moins un hydrocarbure mono-oléfinique.

## Patentansprüche

1. Verfahren zum Regenerieren eines Katalysators zur Dimerisation von Olefinen, der wenigstens teilweise desaktiviert ist und, abgeschieden auf Aluminiumoxid, Chlor und Nickel enthält, dadurch gekennzeichnet, daß es die folgenden Stufen umfaßt:

a) man trocknet diesen Katalysator unter einem Strom wenigstens eines Gases, das gewählt ist aus der Gruppe, die gebildet ist durch molekularen Sauerstoff und ein inertes Gas enthaltendes Gas, bei einer Temperatur zwischen etwa 5 und 70°C ;

b) man kalziniert den aus der Stufe a) stammenden Katalysator durch Erhitzen in nicht-reduzierender Atmosphäre bei einer Temperatur zwischen etwa 300 und 800°C ;

c) man erhitzt den aus der Stufe b) stammenden Katalysator unter oxidierender und chlorierender Atmosphäre bei einer Temperatur zwischen etwa 25 und 750°C ; und

d) man kalziniert den aus der Stufe c) stammenden Katalysator durch Erwärmen in nicht-reduzierender Atmosphäre bei einer Temperatur zwischen etwa 300 und 800°C.

2. Verfahren nach Anspruch 1, dadurch gekennzeichnet, daß es im übrigen nach der Stufe d) eine Stufe umfaßt, in der der aus der Stufe d) stammende Katalysator bis zur Umgebungstemperatur abgekühlt wird.

3. Verfahren nach einem der Ansprüche 1 und 2, bei dem dieser Katalysator, bevor er wenigstens teilweise desaktiviert wird, einen Gewichtsanteil an Nickel zwischen 0,5 und 15% und einen Gewichtsanteil an Chlor zwischen 0,4 und 15% besitzt.

4. Verfahren nach einem der Ansprüche 1 und 2, bei dem dieser Katalysator, bevor er wenigstens teilweise desaktiviert wird, einen Gewichtsanteil an Nickel zwischen 1,5 und 6% und einen Gewichtsanteil an Chlor zwischen 1,3 und 7% besitzt.

5. Verfahren nach einem der Ansprüche 1 bis 4, bei dem die nicht-reduzierende Atmosphäre in der Stufe b) und der Stufe d) auf der Basis eines molekularen Sauerstoff enthaltenden Gasstromes ist.

6. Verfahren nach einem der Ansprüche 1 bis 5, bei dem die oxidierende und chlorierende Atmosphäre in der Stufe c) ein Gasstrom ist, der ein Gemisch wenigstens eines Gases enthält, das molekularen Sauerstoff und wenigstens eine chlorierte Verbindung einschließt.

7. Verfahren nach Anspruch 6, bei dem die chlorierte Verbindung gewählt ist aus der Gruppe, die gebildet ist durch Chlor, Wasserstoffchlorid, Tetrachlorkohlenstoff, Methylchlorid, Dichlorethan, 1,2-Dichlorpropan, Methylenchlorid, Chloroform und die Gemische wenigstens zweier dieser Bestandteile.

8. Verfahren nach einem der Ansprüche 6 und 7, dadurch gekennzeichnet, daß in der Stufe c) man vor dem aus der Stufe b) stammenden Katalysator, bezogen auf den Gasstrom, eine frische Katalysatorcharge gleicher Art anordnet.

9. Verfahren nach Anspruch 8, dadurch gekennzeichnet, daß das Gewichtsverhältnis zwischen der Menge dieses frischen Katalysators und der Menge an zu behandelndem Katalysator zwischen 0,01 und 1 beträgt.

10. Verfahren zur Dimerisation wenigstens eines monoolefinischen Kohlenwasserstoffs umfassend :

a) einen ersten Zeitraum, in flüssiger Phase, innerhalb dessen wenigstens ein Dimerisationskatalysator, der Chlor und Nickel, abgeschieden auf Aluminiumoxid, enthält, mit wenigstens einem monoolefinischen Kohlenwasserstoff unter Dimerisationsbedingungen kontaktiert wird, wobei dieser Zeitraum fortgesetzt wird, bis der Katalysator wenigstens einen Teil seiner Anfangsaktivität verloren hat ;

b) einen zweiten Zeitraum, innerhalb dessen der aus dem Zeitraum a) stammende wenigstens teilweise desaktivierte Katalysator einer Regenerierung ausgesetzt wird, wobei diese Regenerierung nach dem Verfahren eines der Ansprüche 1 bis 9 vorgenommen wird, und

c) einen dritten Zeitraum, innerhalb dessen der aus dem Zeitraum b) stammende Katalysator in den Zeitraum a) zur Kontaktierung mit wenigstens einem monoolefinischen Kohlenwasserstoff zurückgeschickt wird.

## Claims

1. A method for regenerating an olefin dimerization catalyst, at least partially de-activated, containing chlorine and nickel deposited on alumina, characterized in that it comprises the following steps:

a) said catalyst is dried in a stream of at least one gas chosen from the group formed by a gas containing molecular oxygen and an inert gas, at a temperature between about 5 and 70°C ;

b) the catalyst from step a) is calcined by heating in a non reducing atmosphere, at a temperature between about 300 and 800°C ;

c) the catalyst from step b) is heated in an oxidizing and chlorinating atmosphere at a temperature between about 25 and 750°C ;

d) the catalyst from step c) is calcined by heating in a non reducing atmosphere, at a temperature between about 300 and 800°C.

2. The method according to claim 1 characterized in that it further comprises, after step d), a step in which the catalyst from step d) is cooled to room temperature.

3. The method according to one of claims 1 and 2 wherein said catalyst, before being at least partially de-activated, has a nickel content between 0.5 and 15% by weight and a chlorine content between 0.4 and 15% by weight.

4. The method according to one of claims 1 and 2 wherein said catalyst, before being at least partially deactivated, has a nickel content between 1.5 and 6% by weight and a chlorine content between 1.3 and 7% by weight.

5. The method according to one of claims 1 to 4 wherein the non reducing atmosphere in step b) and step d) has as basis a stream of a gas containing molecular oxygen.

6. The method according to one of claims 1 to 5 wherein the oxidizing and chlorinating atmosphere in step c) is a gas stream containing a mixture of at least one gas containing molecular oxygen and at least one chlorinated compound.

7. The method according to claim 6 wherein the chlorinated compound is chosen from the group formed by chlorine, hydrogen chloride, carbon tetrachloride, methyl chloride, dichloroethane, dichloropropane -1,2, methylene chloride, chloroform and mixtures of at least two of these constituents.

8. The method according to one of claims 6 and 7 wherein, in step c), a fresh catalyst charge of the same kind is placed upstream of the catalyst from step b), with respect to the gas stream.

9. The method according to claim 8 wherein the ratio by weight between the amount of said fresh catalyst and the amount of catalyst to be treated is ranging from 0.01 to 1

10. A method for dimerizing at least one mono-olefinic hydrocarbon comprising :

    a) a first period, in the liquid phase, in which at least one dimerization catalyst containing chlorine and nickel deposited on alumina is contacted with at least one mono-olefinic hydrocarbon in dimerization conditions, said period being continued until the catalyst has at least partially lost its initial activity ;

    b) a second period in which the catalyst, at least partially de-activated, coming from period a) is subjected to regeneration, said regeneration being carried out in accordance with the method of one of claims 1 to 9 ;

    c) a third period in which the catalyst from period b) is fed back to period a) where it is contacted with at least one mono-olefinic hydrocarbon.